# EUROPEAN PATENT APPLICATION

(11) **EP 1 329 168 A1**
(43) Date of publication of application: **23.07.2003**
(21) Application number: 01963571.3
(22) Date of filing: 10.09.2001
(51) Int. Cl.: A42B 1/18, A42B 1/06, A42B 3/16

(54) **WINDSHIELD REDUCING WHISTLING SOUNDS OF WIND**

(30) Priority: 03.10.2000 JP 2000342681
(71) Applicant: Mizuno, Ken, Inuyama-shi, Aichi 484-0868 (JP)
(72) Inventor: Mizuno, Ken, Inuyama-shi, Aichi 484-0868 (JP)
(74) Representative: Greenwood, John David
(86) International application number: JP0107859
(87) International publication number: WO02028212

(57) **Abstract**

A windshield covering the vicinity of the front of the ear of a rider on an open-top vehicle. When a person rides on an open-top vehicle with his ears exposed, the air coming from the front dashes against his earlobes, so that the rider hears the whistling sounds of the wind. To reduce these sounds, the rider's earlobes are covered with windshields from the front or from the front to the side. The earlobes are exposed to the open air in the upper, lower and rear regions thereof. This windshield reduces the whistling sounds of the wind. At the same time, the rider sufficiently hears surrounding sounds, and a state in which there is a sufficient amount of heat dissipation from the head is maintained. Thereby, during running, protection against hardness of hearing and effect of improved safety are obtained without feeling hot.

## Description

### Technical Field

The present invention relates to windshields covering the ears of a rider on an open-top vehicle in order to reduce the whistling sounds of wind audible to the rider.

### Technical Background

When a person is riding an open-top vehicle with his ears exposed, he hears the whistling sounds of the wind generated by the riding as the wind dashes against his earlobes. This causes the following three problems:
1. The rider may develop hearing difficulties after riding for a long time.
2. The whistling sounds of the wind may hinder the rider from clearly hearing other sounds coming from the surroundings, thus creating the danger of not being able to judge the situation behind him from what he can hear.
3. For the same reason as given in 2, if the rider is in competition, the whistling sounds may adversely affect the outcome.

On the other hand, if the rider wears a helmet or hat that covers his ears completely, the whistling sounds will be reduced. However, as his ears are isolated from the open air, the rider will have difficulties in hearing other sounds from the surroundings. For the same reason, the amount of heat dissipated from the head decreases so as to make riding in summer unpleasant experience.

A means for facilitating the rider's hearing of surrounding sounds is disclosed in claim 4 of Japan Registered Utility Model No. 3037691. However, as this requires the rider to wear a helmet covering his entire ears, using that means invariably reduces the heat dissipation from the rider's head.

As discussed above, no method existed for reducing the whistling sounds of the wind while maintaining sufficient heat dissipation from the rider's head without diminishing his hearing of surrounding sounds. The present invention provides solution to this problem.

### Disclosure of Invention

Windshields that cover the rider's earlobes from the front or from the front to the side are fitted on the rider's head. The earlobes are exposed to the open air in the upper, lower and rear regions thereof. The windshields are maintained in appropriately intimate contact with the head.

When the windshields are worn by the rider, the wind coming from the front is redirected by the windshields and blows rearward without dashing against the rider's earlobes, thus reducing the whistling sounds of the wind heard by the rider.

The main source of the whistling sounds is turbulence created in the vicinity of the earholes, whereas turbulence at a distance from the earholes contributes less to the problem. Therefore, preventing wind from blowing against the earlobes alone significantly reduces the whistling sounds of the wind. That is, the reduction in the whistling sounds is nearly as effective according to the present invention as in the case of complete covering of the rider's ears.

At the same time, the rider can clearly hear surrounding sounds as his ears are sufficiently exposed to the open air. For the same reason, the heat dissipation from the rider's head does not decrease, so that the rider can wear the invention without feeling hot even in summer.

As described above, the present invention successfully reduces the whistling sounds of the wind while maintaining a sufficient amount of heat dissipation from the head and ensuring sufficient audibility of the surrounding sounds. This provides the following four advantages:
1. The rider does not develop hearing difficulties even after riding for a long time.
2. The rider heals the surrounding sounds more easily. Accordingly, he can judge the situation behind him through sound, thus improving the safety in riding.
3. If the rider is in competition, his chance of victory improves for the reason given in 2.
4. The advantages described in 1-3 are provided without the sensation of heat.

### Brief Description Of The Attached Drawings

All the drawings show embodiments of the invention.

Figure 1 is an illustration of Embodiment 1 seen from an upper front left perspective, showing a single windshield unit. Figure 2 is a left side elevation of Embodiment 1, showing a rider wearing the unit.

Figure 3 is an illustration of Embodiment 2 seen from an upper front left perspective, showing the left windshield. Figure 4 is a left side elevation of Embodiment 2, showing a rider wearing the windshields in combination with a helmet.

Figure 5 is a left side elevation of Embodiment 3-1, showing a rider wearing the windshields in combination with a helmet.

Figure 6 is an illustration of Embodiment 3-2 as seen from an upper front left perspective, showing a single helmet.

The following is a list of the legends used herein:
1: Main windshield body
2: Support
3: Cushioning material
4: Rider's earlobe
5: Through-hole
6: Chin strap of helmet
7: Stopper
8: Head covering of helmet
9: Head covering extension
10: Auxiliary windshield

### Best Mode For Carrying Out The Invention

The following three embodiments of windshields to be fitted on the rider are considered:
1. A windshield unit having a support directly fitted on the rider's head.
2. Windshields attached to a helmet, a headband, eyeglasses, or other items worn on the head.
3. A helmet modified to incorporate the function of a windshield in the modified parts.

These three embodiments will be described hereinafter with reference to the attached drawings.

### Embodiment 1

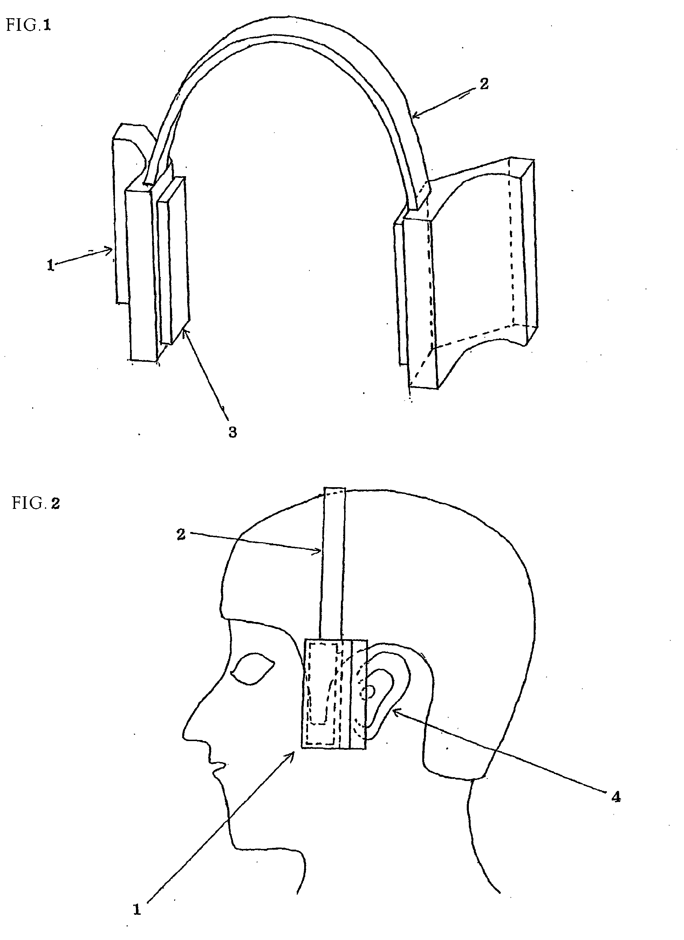

Figures 1 and 2 show an example of a windshield unit with an arcuate support directly fitted on a rider's head. The holding force of the support 2 secures the windshields 1 to the head of the rider not wearing a helmet. The holding force of the support 2 and the effect of the cushioning material 2 maintain the windshields 1 in intimate contact with the head.

Although omitted from the drawings, a mechanism for adjusting the size of the support to the rider's head is preferably incorporated in the support. In order to ensure safety for the rider, the holding force is preferably set to the minimum required for normal wearing conditions such that, in the event of an accident, the entire windshield unit is allowed to easily come off the head by the impact.

### Embodiment 2

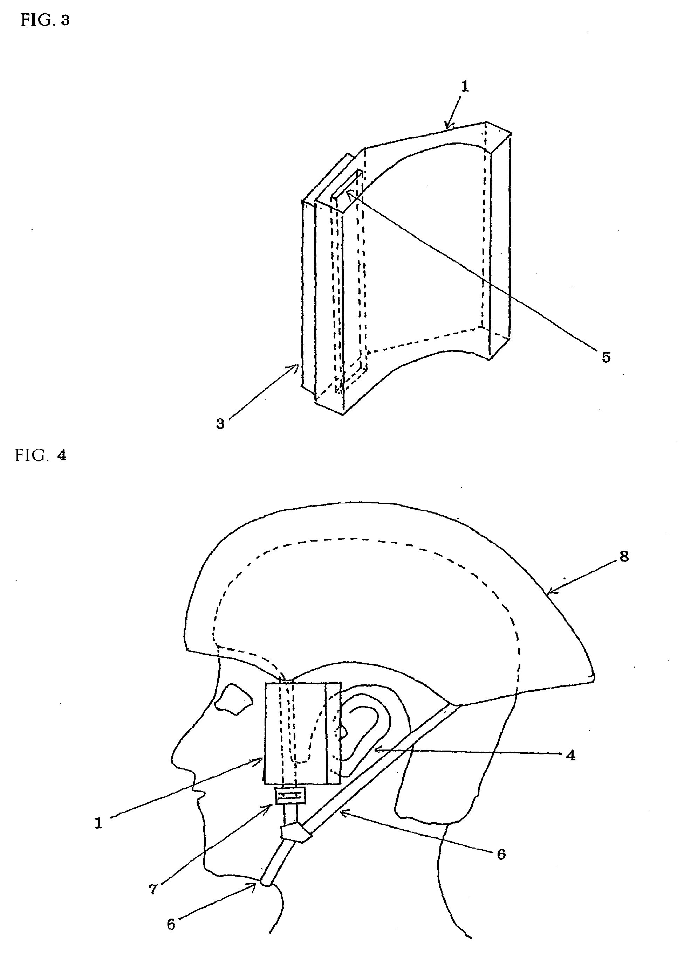

Figures 3 and 4 show an example in which windshields are attached to the chin strap of a helmet. The chin strap 6 is passed through the holes 5 provided in the windshields 1. The tensile force of the chin strip 6 and the effect of the cushioning material 2 maintain the windshields in intimate contact with the head. The stoppers 7 support the windshields 1, securely maintaining them in optimum positions.

### Embodiment 3-1

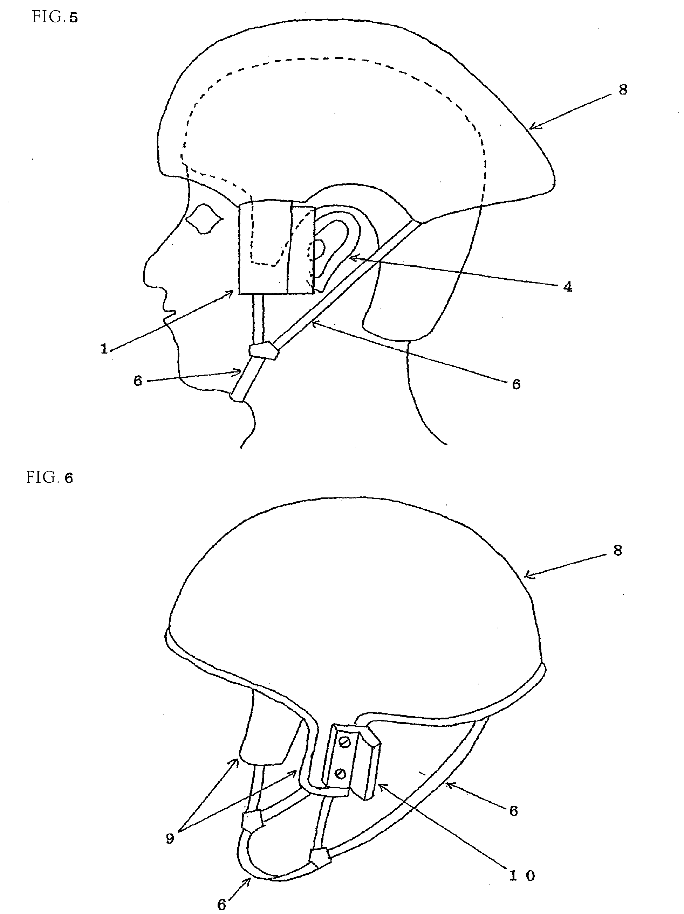

Figure 5 shows an example in which the chin strap of a helmet is modified to incorporate the functionality of windshields. This embodiment incorporates the windshields of Embodiment 2 into the chin strap during the manufacturing stage of the helmet.

### Embodiment 3-2

Figure 6 shows an example in which the head covering of a helmet is partially modified to incorporate the functionality of windshields in the modified portions. Specifically, the head covering of the helmet has downward extensions in front of the wearer's ears so as to provide a windshield function to the head covering extensions 9. If the whistling sounds of the wind are not sufficiently reduced by the covering extensions alone, auxiliary windshields 10 are added to further enhance the muffling effect.

Many modifications are possible other than those shown above. For example, Embodiment 1 may be modified to provide a hook-shaped support to each windshield that permits attachment thereof to the rider's earlobe. The windshields of Embodiment 2 may be attached to eyeglasses or a headband.

### Industrial Applicability

The invention is intended chiefly for incorporation with bicycle, motorcycle, and horseback riding helmets. Most of bicycle and horseback riding helmets and some motorcycle helmets are designed to completely expose riders' ears for coolness and lightness. Embodiments 2 and 3 of the present invention effectively reduce the whistling sounds of wind while achieving these objectives.

Embodiment 1 is suitable for use with high-speed vehicles on which helmets are not customarily worn, such as water skies and personal watercraft.

## Claims

1. A windshield for reducing the whistling sounds of wind, the windshield being **characterized in** covering the earlobe of a rider on an open-top vehicle from the front or from the front to the side of the earlobe while exposing the earlobe to the open air in the upper, lower and rear regions thereof.
